# EUROPEAN PATENT SPECIFICATION

(11) **EP 3 740 216 B1**
(45) Date of publication and mention of the grant of the patent: **02.03.2022**
(21) Application number: 19705845.6
(22) Date of filing: 17.01.2019
(51) Int. Cl.: A61K 35/17, C12N 5/10, C12N 15/867, A61P 35/00

(54) **METHODS OF ASSESSING TRANSDUCTION POTENCY OF VIRAL VECTORS**
METHODEN ZUR BEWERTUNG DER TRANSDUKTIONSPOTENZ VON VIRALEN VEKTOREN
PROCÉDÉS D'ÉVALUATION DE LA POTENCE DE TRANSDUCTION DE VECTEURS VIRAUX

(30) Priority: 17.01.2018 US 201862618295 P; 17.01.2018 DE 102018100967
(43) Date of publication of application: 25.11.2020
(73) Proprietor: Immatics US, Inc., Houston, TX 77030 (US)
(72) Inventor: KALRA, Mamta, Houston, TX 77077 (US); BOURGOGNE, Agathe, Houston, TX 77035 (US); MOHAMED, Ali, Sugar Land, TX 77479 (US); WALTER, Steffen, Houston, TX 77005 (US)
(74) Representative: Zwicker, Jörk
(86) International application number: PCT/US2019/013939
(87) International publication number: WO 2019/143772

(56) References cited:
- BRUCE L. LEVINE ET AL: "Global Manufacturing of CAR T Cell Therapy", MOLECULAR THERAPY - METHODS & CLINICAL DEVELOP, vol. 4, 4 March 2017 (2017-03-04), pages 92-101, XP055510414, GB ISSN: 2329-0501, DOI: 10.1016/j.omtm.2016.12.006
- CRIBBS A.P. ET AL: "Simplified production and concentration of lentiviral vectors to achieve high transduction in primary human T cells", BMC BIOTECHNOLOGY, vol. 13, no. 1, 12 November 2013 (2013-11-12), page 98, XP021167925,
- Verhoyen E. et al.: "Chapter 8: Lentiviral vector gene transfer into human T cells." In: Baum C (ed.): "Methods in Molecular Biology, Methods and Protocols", 2009, Humana Press, XP002790298, vol. 506, pages 97-114, page 97 - page 114
- GAY V. ET AL: "Quantification of HIV-based lentiviral vectors: influence of several cell type parameters on vector infectivity", ARCHIVES OF VIROLOGY, vol. 157, no. 2, 29 October 2011 (2011-10-29), pages 217-223, XP035010306,

## Description

### BACKGROUND

### 1. Field

The present disclosure relates to a method of making T cells suitable for immunotherapy.

### 2. Background

A challenge in the delivery of a gene by a viral vector or a virus for therapeutic purposes is the preparation and accurate quantification of clinical dosage forms. The production of viral vaccines, recombinant proteins using viral vectors and viral antigens all require virus quantification to continually adapt and monitor the process in order to optimize production yields and respond to the ever-changing demands and applications.

Virus titer determination or virus quantification involves counting the number of viruses in a specific volume to determine the virus concentration. Traditional methods include viral plaque assays, which determine the number of plaque forming units (pfu) in a virus sample and the Tissue Culture Infective Dose (TCID₅₀) or Fluorescence Active Infectious Dose (FAID₅₀) which measures the infectious virus titer. This TCID₅₀ assay quantifies the amount of virus required to kill 50% of infected hosts or to produce a cytopathic effect in 50% of inoculated tissue culture cells. The traditional methods are generally slow and labor-intensive, and suffer from limitations including a high degree of inter-assay variability.

Enzyme-Linked Immunosorbent Assay (ELISA) is a more modern variation of a protein assay that utilizes a specific antibody linked to an enzyme to detect the presence of an unknown amount of antigen (i.e. virus) in a sample. The antibody-antigen binding event is detected and/or quantified through the enzyme's ability to convert a reagent to a detectable signal that can be used to calculate the concentration of the antigen in the sample. The plate assays of the virus titer determination that are based on immunofluorescence detection using an ELISA are developed, however they are used to quantify proteins from virus samples and not to quantify infectious viruses.

Flow cytometry or FACS (fluorescence-activated cell sorter) assays have been used to measure the number of infected cells in cell cultures infected at relatively high multiplicities of infection (MOI). For example, U.S. Pat. No. 6,248,514 describe the use of flow cytometry to analyze cells infected using specified ranges of viral particle concentration and adsorption time yields. U.S. Pat. No. 7,476,507 describe FACS-based methods for the determination of the viral titer of a culture of host animal host cells infected with a circovirus. However, for many applications, the cost, size and complexity of the flow cytometry instruments prevent wider use.

Methods of evaluating retroviral vector titers can generally be divided into functional and non-functional titration methods. Non-functional titration methods may include p24 antigen ELISA, assessment of reverse transcriptase (RT) activity, and determination of genomic RNA concentration in vector preparations by semi-quantitative northern blotting, dot blot analysis, or RT-qPCR. Sometimes these techniques overestimate the functional vector titer and suffer from certain disadvantages. For example, p24 protein pool quantified may include a variable amount of free p24 and p24 of non-functional vector particles. Similarly, RNA titers may assess defective particles, whereas the RT-assay may demonstrate RT activity. More accurate functional titers may be determined by transduction of cells following limiting dilution of vector and subsequent evaluation of reporter protein activity, e.g., β-galactosidase positive cells, or by assessment of the number of colony forming units following antibiotic selection. More widespread and straightforward techniques to quantify functional vector titers may use eGFP fluorescence and fluorescence-activated cell sorting (FACS). However, FACS analysis of transgene expression may be restricted to fluorescent reporter proteins and may not discriminate between cells with single or multiple integrations.

US20170166866 describes a method of transducing a primary T lymphocyte, including contacting a primary T lymphocyte with a viral vector, e.g., lentiviral vector, containing a nucleic acid at a multiplicity of infection (MOI) of 1.5 to 2.5 and a compound that is an inhibitor of the innate immune system, so that the nucleic acid is transduced into T lymphocyte.

US20170023570 describes a high throughput method of quantitating infectious viral particles in a sample. This method of virus quantification includes the steps of: 1) providing a sample containing a virus; 2) preparing serial dilutions of the sample; 3) infecting host cells with the virus and incubating the culture; 4) reacting an antigen expressed by the virus in infected cells with an antibody labelled with a fluorescent tag; 5) determining the number of infected cells; and 6) determining the virus titer in the sample. A need remains to develop better methods for assessing optimal viral concentrations for viral transduction in manufacturing T cells for immunotherapy.

Levine et al., 2017 (Mol. Therapy - Methods & Clin. Development, Vol. 4:92-101), Verhoyen et al., 2009 (Methods in Mol. Biol., Methods & Protocols, Chapter 8) and Cribbs et al., 2013 (BMC Biotechnology, Vol. 13(98) disclose methods for preparing engineered T cells obtained from patients for being used in immunotherapy. However, none of the methods teaches how to determine the optimal amount of virus to be added for transduction.

There is a need in the field for accurate methods of quantifying viral particles in manufacturing T cell products.

### BRIEF SUMMARY

In an aspect, the disclosure provides for methods of making T cells suitable for immunotherapy, comprising:
(A1) obtaining primary T cells from at least one healthy individual;
(A2) activating the T cells obtained in step (A1) with an anti-CD3 antibody and an anti-CD28 antibody;
(A3) transducing the activated T cells of step (A2) with a with a transduction mixture comprising a cell concentration of 0.1 × 10⁶ cells/ml to 1.0 × 10⁸ cells/ml and a retroviral vector at a plurality of volumetric concentrations; viz. volume of virus sample per volume of transducing mixture, wherein the retroviral vector carries a transgene;
(A4) expanding the transduced T cells of step (A3); and
(A5) identifying the volumetric concentration of the retroviral vector that yields a maximum average of the quantity of the expanded T cells that express the transgene and/or a maximum average of the copy number of the integrated transgene without exceeding five copies of the integrated transgene in each of the expanded T cells from step (A1); and(B1) obtaining primary T cells from a patient;
(B2) activating the T cells obtained in step (B1) with a transduction mixture comprising a cell concentration of 0.1 × 10⁶ cells/ml to 1.0 × 10⁸ cells/ml and the retrovirus used in step (A3) an anti-CD3 antibody and an anti-CD28 antibody (B3) transducing the activated T cells of step (B2) with a transduction mixture comprising a cell concentration of 0.1 × 10⁶ cells/ml to 1.0 × 10⁸ cells/ml at the retroviral volumetric concentration identified in step (A5);
(B4) expanding the transduced primary T cells of step (B3).

In another aspect, the retroviral vector is a retroviral vector expressing a T cell receptor (TCR).

In yet another aspect, the retroviral vector is a lentiviral vector expressing a TCR.

In another aspect, the transduction mixture may contain a cell concentration of from about 0.1 × 10⁶ cells/ml to about 1.0 × 10⁶ cells/ml, from about 0.5 × 10⁶ cells/ml to about 1.0 × 10⁶ cells/ml, from about 1.0 × 10⁶ cells/ml to about 1.0 × 10⁷ cells/ml, from about 5.0 × 10⁶ cells/ml to about 1.0 × 10⁷ cells/ml, from about 1.0 × 10⁷ cells/ml to about 1.0 × 10⁸ cells/ml, or from about 5.0 × 10⁷ cells/ml to about 1.0 × 10⁸ cells/ml.

In an aspect, the T cells in step (A1) are obtained from a plurality of healthy individuals.

In an aspect, the patient or individual in need thereof is a cancer patient. In another aspect, the cancer to be treated is selected from the group consisting of hepatocellular carcinoma (HCC), colorectal carcinoma (CRC), glioblastoma (GB), gastric cancer (GC), esophageal cancer, non-small cell lung cancer (NSCLC), pancreatic cancer (PC), renal cell carcinoma (RCC), benign prostate hyperplasia (BPH), prostate cancer (PCA), ovarian cancer (OC), melanoma, breast cancer, chronic lymphocytic leukemia (CLL), Merkel cell carcinoma (MCC), small cell lung cancer (SCLC), Non-Hodgkin

In another aspect, the T cells obtained in step (A1) and step (B1) are CD8⁺ T cells.

Furher aspects and embodiments of the present invention are disclosed in the accompanying set of claims.

### BRIEF DESCRIPTION OF THE DRAWINGS

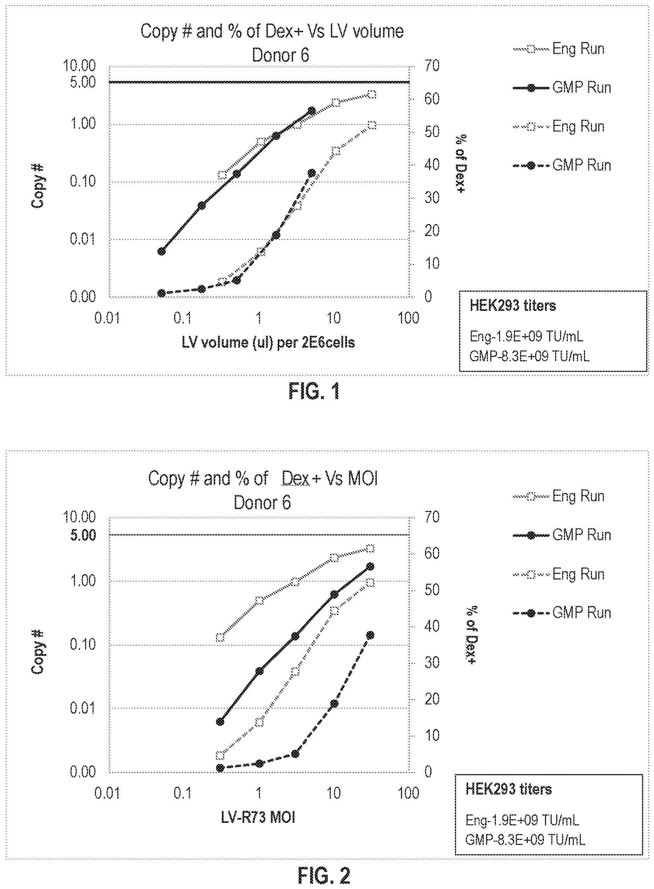

FIG. 1 shows similar copy number of integrated viral vector (lentivirus (LV)-R73) (solid lines) and quantity (% of MHC Dextramer (Dex)+) of T cells that express the transgene (R7P1 D5 TCR) (dotted lines) in T cells obtained from a healthy donor # 6 in engineering (Eng Run) and GMP (GMP Run) batches when aligned based on volumetric concentrations.
FIG. 2 shows different copy number of integrated viral vector (LV-R73) (solid lines) and quantity (% of Dex+) of T cells that express the transgene (R7P1 D5 TCR) (dotted lines) in T cells obtained from a healthy donor # 6 in engineering (Eng Run) and GMP (GMP Run) batches when aligned based on multiplicities of infection (MOI).

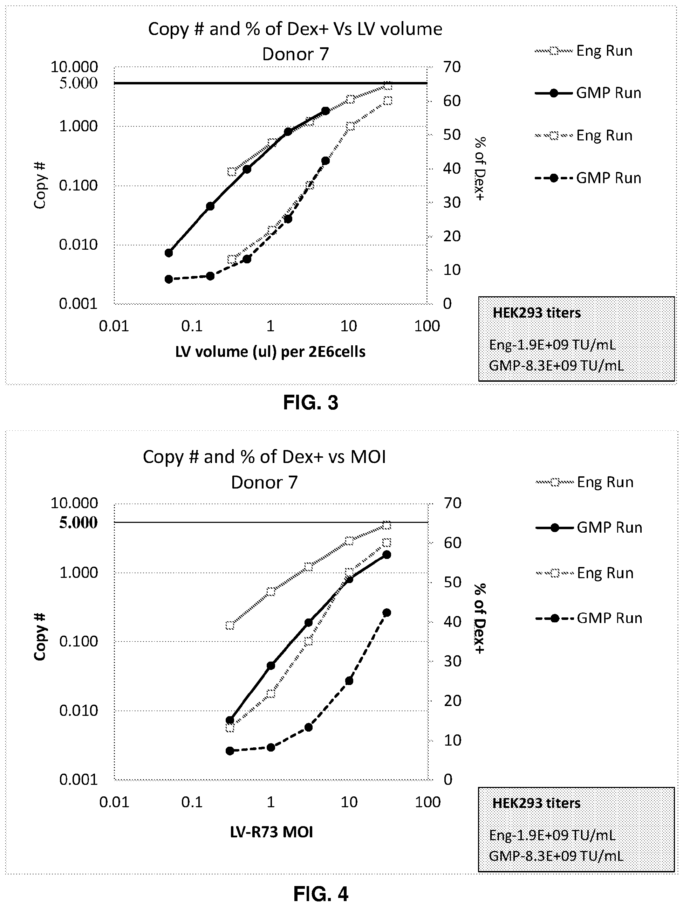

FIG. 3 shows similar copy number of integrated viral vector (LV-R73) (solid lines) and quantity (% of Dex+) of T cells that express the transgene (R7P1 D5 TCR) (dotted lines) in T cells obtained from a healthy donor # 7 in engineering (Eng Run) and GMP (GMP Run) batches when aligned based on volumetric concentrations.
FIG. 4 shows different copy number of integrated viral vector (LV-R73) (solid lines) and quantity (% of Dex+) of T cells that express the transgene (R7P1 D5 TCR) (dotted lines) in T cells obtained from a healthy donor # 7 in engineering (Eng Run) and GMP (GMP Run) batches when aligned based on multiplicities of infection (MOI).

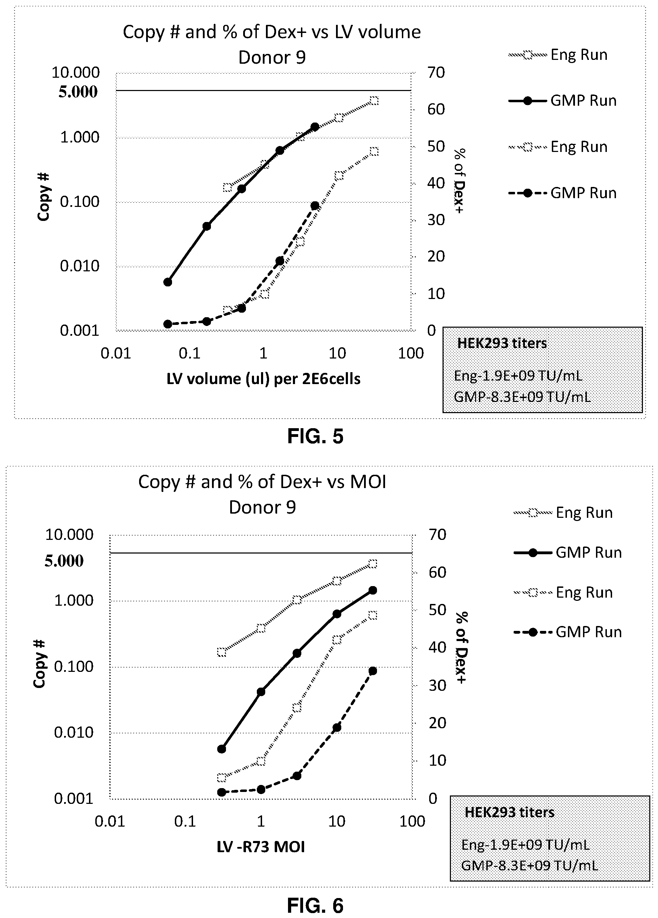

FIG. 5 shows similar copy number of integrated viral vector (LV-R73) (solid lines) and quantity (% of Dex+) of T cells that express the transgene (R7P1 D5 TCR) (dotted lines) in T cells obtained from a healthy donor # 9 in engineering (Eng Run) and GMP (GMP Run) batches when aligned based on volumetric concentrations.
FIG. 6 shows different copy number of integrated viral vector (LV-R73) (solid lines) and quantity (% of Dex+) of T cells that express the transgene (R7P1 D5 TCR) (dotted lines) in T cells obtained from a healthy donor # 9 in engineering (Eng Run) and GMP (GMP Run) batches when aligned based on multiplicities of infection (MOI).

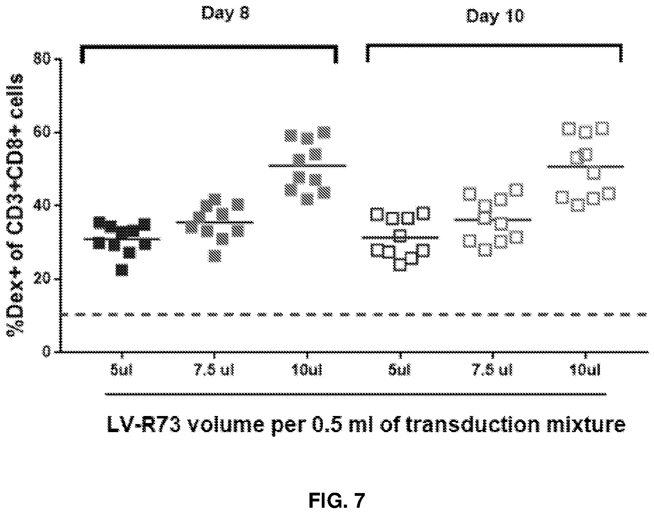

FIG. 7 shows quantity (% Dex+ of CD3+CD8+ cells) of T cells that express the transgene (R7P1D5 TCR) in T cells obtained from 10 healthy donors transduced with viral vector at three selected volumetric concentrations to select the optimal virus volume.

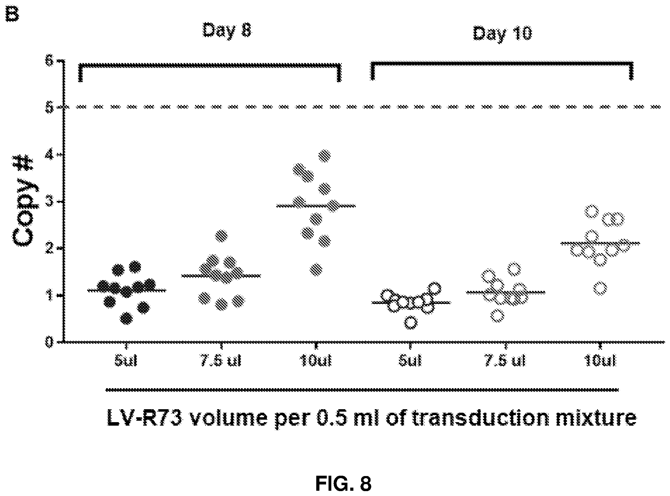

FIG. 8 shows copy numbers of integrated viral vector (LV-R73) in T cells obtained from 10 healthy donors transduced with viral vector at three selected volumetric concentrations to select the optimal virus volume.

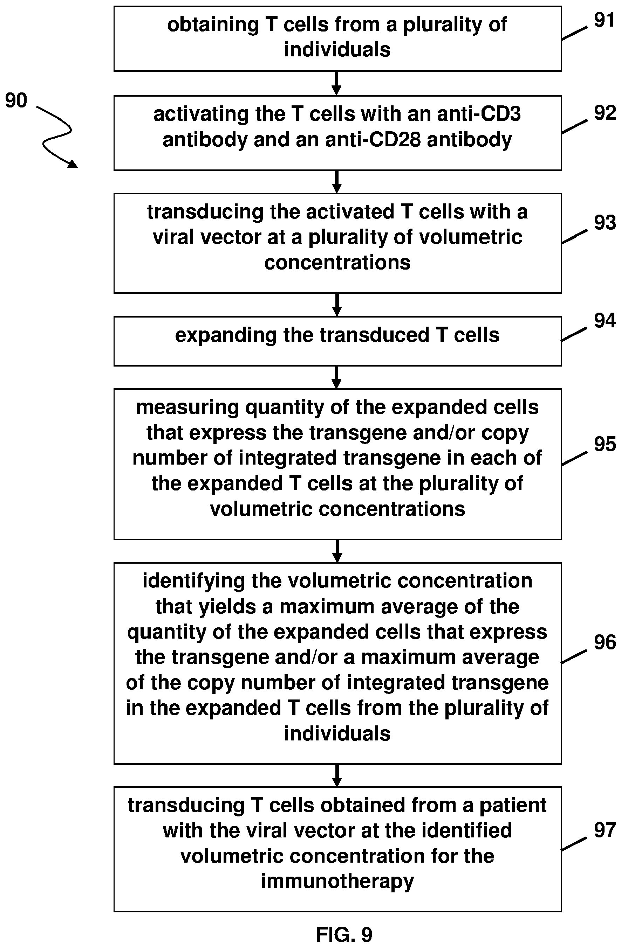

FIG. 9 illustrates a method in accordance with one embodiment of the present disclosure.

### DETAILED DESCRIPTION

Disclosed are T cells transduced with a viral vector at a volumetric concentration and methods thereof. In an aspect, the present disclosure provides for the assessment of optimal lentiviral vector concentrations for transducing T cells.

Also disclosed are T cell populations produced by methods described herein.

In an aspect, the present disclosure comprises a method of making T cells suitable for immunotherapy, the method comprising:
(A1) obtaining primary T cells from at least one healthy individual;
(A2) activating the T cells obtained in step (a1) with an anti-CD3 antibody and an anti-CD28 antibody;
(A3) transducing the activated T cells of step (A2) with a with a transduction mixture comprising a cell concentration of 0.1 × 10⁶ cells/ml to 1.0 × 10⁸ cells/ml and a retroviral vector at a plurality of volumetric concentrations; viz. volume of virus sample per volume of transducing mixture, wherein the retroviral vector carries a transgene; and
(A4) expanding the transduced T cells of step (A3); and
(A5) identifying the volumetric concentration of the retroviral vector that yields a maximum average of the quantity of the expanded T cells that express the transgene and/or a maximum average of the copy number of the integrated transgene without exceeding five copies of the integrated transgene in each of the expanded T cells from step (A1); and(B1) obtaining primary T cells from a patient;
(B2) activating the T cells obtained in step (B1) with an anti-CD3 antibody and an anti-CD28 antibody;
(B3) transducing the activated T cells of step (B2) with a transduction mixture comprising a cell concentration of 0.1 × 10⁶ cells/ml to 1.0 × 10⁸ cells/ml at the retroviral volumetric concentration identified in step (A5);
(B4) expanding the transduced primary T cells of step (B3).

T cells can be obtained from a plurality of healthy donors, patients, or individuals. The T cells can be obtained from one or more, two or more, three or more, four or more, five or more, ten or more, or 20 or more healthy donors, patients, or individuals.

In another aspect, the retroviral vector is a retroviral vector expressing a T cell receptor (TCR).

The volumetric concentrations can be from about 0.01 µl per about 10⁶ cells to about 1 ml per about 10⁶ cells; from about 0.01 µl per about 2 × 10⁶ cells to about 1 ml per about 2 × 10⁶ cells; from about 0.01 µl per about 5 × 10⁶ cells to about 1 ml per about 5 × 10⁶ cells; from about 0.01 µl per about 10⁷ cells to about 1 ml per about 10⁷ cells; from about 1 µl per about 10⁷ cells to about 500 µl per about 10⁷ cells; from about 5 µl per about 10⁷ cells to about 150 µl per about 10⁷ cells; or from about 8 µl per about 10⁷ cells to about 12 µl per about 10⁷ cells.

As used herein, the term "about" is defined as ±5% of the recited value.

The term "volumetric concentration" used herein refers to volume, e.g., ml and µl, of virus used per volume, e.g., ml and µl, of transduction mixture (media or diluent). The volume of transduction mixture may be determined by the cell concentration during transduction. For example, if cell concentration during transduction is fixed at 2.0 × 10⁶ cells/ml, then 2.0 × 10⁶ transduced cells may be in a fixed volume of 1.0 ml of transduction mixture or 1.0 × 10⁶ transduced cells may be in a fixed volume of 0.5 ml of transduction mixture.

The methods described herein may include identifying the volumetric concentration that yields a maximum average of the quantity of the expanded T cells that express the transgene in the expanded T cells from the plurality of healthy donors, e.g., measuring % of cells positive for the transgene expression.

The method may includes identifying the volumetric concentration that does not exceed the maximum average copy number of 5 copies of the integrated transgene in the expanded T cells from the plurality of healthy donors.

In an aspect, the patient or individual is a cancer patient. In another aspect, a cancer described herein is selected from the group consisting of hepatocellular carcinoma (HCC), colorectal carcinoma (CRC), glioblastoma (GB), gastric cancer (GC), esophageal cancer, non-small cell lung cancer (NSCLC), pancreatic cancer (PC), renal cell carcinoma (RCC), benign prostate hyperplasia (BPH), prostate cancer (PCA), ovarian cancer (OC), melanoma, breast cancer, chronic lymphocytic leukemia (CLL), Merkel cell carcinoma (MCC), small cell lung cancer (SCLC), Non-Hodgkin lymphoma (NHL), acute myeloid leukemia (AML), gallbladder cancer and cholangiocarcinoma (GBC, CCC), urinary bladder cancer (UBC), acute lymphoblastic leukemia (ALL), and uterine cancer (UEC).

Also disclosed are vectors. A "vector" can be capable of transferring gene sequences to target cells. Typically, "vector construct," "expression vector," and "gene transfer vector," meaning any nucleic acid construct capable of directing the expression of a gene of interest and which can transfer gene sequences to target cells. Thus, the term includes cloning and expression vectors, as well as integrating vectors.

Retroviral vectors have been designed based on various members of the *Retroviridae* including Foamyvirus, Human Immunodeficiency Virus (HIV-1), Simian Immunodeficiency Virus (SIV), Bovine Immunodeficiency Virus, Feline Immunodeficiency Virus, Equine Infectious Anemia Virus (EIAV), Murine Leukemia Virus (MLV), Bovine Leukemia Virus, Rous Sarcoma Virus (RSV), Spleen Necrosis Virus (SNV), and Mouse Mammary Tumor Virus. Commonly used platforms may include Foamyvirus-derived and HIV-1 derived lentiviral vectors, and gammaretroviral vectors derived from MLV. The tropism of a retrovirus can be altered by incorporating foreign envelope proteins, expanding the potential target population of target cells. For example, incorporation of vesicular stomatitis virus G glycoprotein (VSV-G) envelope protein may broaden the tropism and allow gene transfer into a broad variety of cells *in vitro, e.g.,* CD34+ stem cells, and *in vivo,* e.g., brain, muscle, and liver. Incorporation of Baculovirus GP64 and hepatitis C E1 and E2 pseudotyping envelope proteins may enhance hepatic transduction and incorporation of RD114 pseudotyping envelope protein may favor transduction in lymphohematopoietic cells. Lentiviral vectors can transduce or infect non-dividing cells and typically produce high viral titers. Selection of a lentiviral or a gammaretroviral gene transfer system depends on the target tissue. These vectors may be comprised of cis-acting long terminal repeats with packaging capacity for up to 6-10 kb of foreign sequence. The minimum cis-acting LTRs are sufficient for replication and packaging of the vectors, which are then used to integrate the therapeutic gene into the target cell to provide permanent transgene expression.

In certain embodiments, the vector is a lentiviral vector expressing a TCR. A lentiviral vector, as used herein, is a vector which comprises at least one component part derivable from a lentivirus. A detailed list of lentiviruses may be found in Coffin et al. (1997) "Retroviruses" Cold Spring Harbour Laboratory Press Eds: J M Coffin, S M Hughes, H E Varmus pp 758-763). Lentiviral vectors can be produced by methods. See, e.g., U.S. Pat. Nos. 5,994,136; 6,165,782; and 6,428,953. Preferably, the lentiviral vector is an integrase deficient lentiviral vector (IDLV). See, e.g., U.S. Patent Publication 2009/0117617. IDLVs may be produced as described, for example using lentivirus vectors that include one or more mutations in the native lentivirus integrase gene, for instance, as disclosed in Leavitt et al. (1996) J. Virol. 70(2):721-728; Philippe et al. (2006) Proc. Natl Acad. Sci USA 103(47): 17684-17689; and WO 06/010834. The IDLV is preferably an HIV lentiviral vector comprising a mutation at position 64 of the integrase protein (D64V), as described in Leavitt et al. (1996) J. Virol. 70(2):721-728.

In gene therapy applications, it may be desirable that the gene therapy vector be delivered with a high degree of specificity to a particular tissue type. Accordingly, a viral vector can be modified to have specificity for a given cell type by expressing a ligand as a fusion protein with a viral coat protein on the outer surface of the virus. The ligand is chosen to have affinity for a receptor known to be present on the cell type of interest. For example, Han et al., Proc. Natl. Acad. Sci. USA 92:9747-9751 (1995), reported that Moloney murine leukemia virus can be modified to express human heregulin fused to gp70, and the recombinant virus infects certain human breast cancer cells expressing human epidermal growth factor receptor. This principle can be extended to other virus-target cell pairs, in which the target cell expresses a receptor and the virus expresses a fusion protein comprising a ligand for the cell-surface receptor. For example, filamentous phage can be engineered to display antibody fragments (e.g., FAB or Fv) having specific binding affinity for virtually any chosen cellular receptor. Although the above description applies primarily to viral vectors, the same principles can be applied to non-viral vectors. Such vectors can be engineered to contain specific uptake sequences which favor uptake by specific target cells.

Gene therapy vectors can be delivered in vivo by administration to an individual patient, typically by systemic administration (e.g., intravenous, intraperitoneal, intramuscular, subdermal, or intracranial infusion) or topical application, as described below. Alternatively, vectors can be delivered to cells ex vivo, such as cells explanted from an individual patient (e.g., lymphocytes, bone marrow aspirates, tissue biopsy) or universal donor hematopoietic stem cells, followed by re-implantation of the cells into a patient, usually after selection for cells which have incorporated the vector.

Suitable cells include, but are not limited to, eukaryotic and prokaryotic cells and/or cell lines. Non-limiting examples of such cells or cell lines generated from such cells include COS, CHO (e.g., CHO-S, CHO-K1, CHO-DG44, CHO-DUXB11, CHO-DUKX, CHOK1SV), VERO, MDCK, WI38, V79, B14AF28-G3, BHK, HaK, NS0, SP2/0-Ag14, HeLa, HEK293 (e.g., HEK293-F, HEK293-H, HEK293-T), and perC6 cells, as well as insect cells such as Spodoptera fugiperda (Sf), or fungal cells such as Saccharomyces, Pichia and Schizosaccharomyces. The cell line is preferably a CHO-K1, MDCK or HEK293 cell line. Additionally, primary cells may be isolated and used ex vivo for reintroduction into the subject to be treated following treatment with the nucleases (e.g., ZFNs or TALENs) or nuclease systems (e.g., CRISPR/Cas). Suitable primary cells include peripheral blood mononuclear cells (PBMC), and other blood cell subsets such as, but not limited to, T-lymphocytes such as CD4+ T cells or CD8+ T cells. Suitable cells also include stem cells such as, by way of example, embryonic stem cells, induced pluripotent stem cells, hematopoietic stem cells (CD34+), neuronal stem cells and mesenchymal stem cells. Vectors suitable for introduction of transgenes into immune cells (e.g., T cells) include nonintegrating lentivirus vectors.

In conventional manufacturing of T cells, quantity of viral vector to be used is oftentimes based on the titer of the viral batch determined on a cell line like 293T. Using this titer, virus is tested over a range of multiplicity of infection (MOI), which, when referring to a group of cells inoculated with virus particles, is the ratio of the number of virus particles to the number of target cells present in a defined space. The highest multiplicities of infection (MOI) value in the linear range may be selected as the optimal multiplicities of infection (MOI). However, this method may allow wide room for variation due to the use of a tumor cell line, e.g., 293T, for determination of titer and a titer dependent variable multiplicities of infection (MOI). Disclosed herein are methods for determining optimal virus volume used in manufacturing and assessing the potency of different viral vector batches. For example, rather than using cell lines, e.g., 293T cells, primary human T cells from healthy donors may be used by following the same T cell manufacturing process in smallscale, e.g., 1-2 million cells in a well of 24 well G-Rex (2 cm²), mid-scale may have, e.g., 5 million cells in a well of 6 well G-Rex (10 cm²), or large-scale may have 50 million cells in a G-Rex 100 (100 cm²). Good Manufacturing Process (GMP) scale may start with 250-400 million cells in 5-8 G-Rex100. The read outs obtained from different scales may be directly relevant to clinical manufacturing.

As such, methods of the present disclosure may be more robust than conventional methods using cell lines derived titers and multiplicities of infection (MOI) and may reduce variation when multiple lentiviral vector batches are required for the manufacturing of the same T cell product

An "exogenous" molecule is a molecule that is not normally present in a cell, but can be introduced into a cell by one or more genetic, biochemical or other methods. "Normal presence in the cell" is determined with respect to the particular developmental stage and environmental conditions of the cell. Thus, for example, a molecule that is present only during embryonic development of muscle is an exogenous molecule with respect to an adult muscle cell. Similarly, a molecule induced by heat shock is an exogenous molecule with respect to a non-heat-shocked cell. An exogenous molecule can comprise, for example, a functioning version of a malfunctioning endogenous molecule or a malfunctioning version of a normally-functioning endogenous molecule.

An exogenous molecule can be, among other things, a small molecule, such as is generated by a combinatorial chemistry process, or a macromolecule such as a protein, nucleic acid, carbohydrate, lipid, glycoprotein, lipoprotein, polysaccharide, any modified derivative of the above molecules, or any complex comprising one or more of the above molecules. Nucleic acids include DNA and RNA, can be single- or double-stranded, can be linear, branched or circular, and can be of any length. Nucleic acids include those capable of forming duplexes, as well as triplex-forming nucleic acids. See, for example, U.S. Pat. Nos. 5,176,996 and 5,422,251. Proteins include, but are not limited to, DNA-binding proteins, transcription factors, chromatin remodeling factors, methylated DNA binding proteins, polymerases, methylases, demethylases, acetylases, deacetylases, kinases, phosphatases, integrases, recombinases, ligases, topoisomerases, gyrases and helicases.

An exogenous molecule can be the same type of molecule as an endogenous molecule, e.g., an exogenous protein or nucleic acid. For example, an exogenous nucleic acid can comprise an infecting viral genome, a plasmid or episome introduced into a cell, or a chromosome that is not normally present in the cell. Methods for the introduction of exogenous molecules into cells are known to those of skill in the art and include, but are not limited to, lipid-mediated transfer (i.e., liposomes, including neutral and cationic lipids), electroporation, direct injection, cell fusion, particle bombardment, calcium phosphate coprecipitation, DEAE-dextran-mediated transfer and viral vector-mediated transfer.

In contrast, an "endogenous" molecule is one that is normally present in a particular cell at a particular developmental stage under particular environmental conditions. For example, an endogenous nucleic acid can comprise a chromosome, the genome of a mitochondrion, chloroplast or other organelle, or a naturally-occurring episomal nucleic acid. Additional endogenous molecules can include proteins, for example, transcription factors and enzymes.

A "gene," for the purposes of the present disclosure, includes a DNA region encoding a gene product (see infra), as well as all DNA regions which regulate the production of the gene product, whether or not such regulatory sequences are adjacent to coding and/or transcribed sequences. Accordingly, a gene includes, but is not necessarily limited to, promoter sequences, terminators, translational regulatory sequences such as ribosome binding sites and internal ribosome entry sites, enhancers, silencers, insulators, boundary elements, replication origins, matrix attachment sites and locus control regions.

"Gene expression" refers to the conversion of the information, contained in a gene, into a gene product. A gene product can be the direct transcriptional product of a gene (e.g., mRNA, tRNA, rRNA, antisense RNA, ribozyme, structural RNA or any other type of RNA) or a protein produced by translation of a mRNA. Gene products also include RNAs which are modified by processes such as capping, polyadenylation, methylation, and editing, and proteins modified by, for example, methylation, acetylation, phosphorylation, ubiquitination, ADP-ribosylation, myristilation, and glycosylation.

"Modulation" of gene expression refers to a change in the activity of a gene. Modulation of expression can include, but is not limited to, gene activation and gene repression. Modulation may also be complete, i.e., wherein gene expression is totally inactivated or is activated to wild-type levels or beyond; or it may be partial, wherein gene expression is partially reduced, or partially activated to some fraction of wildtype levels.

The terms "nucleic acid," "polynucleotide," and "oligonucleotide" are used interchangeably and refer to a deoxyribonucleotide or ribonucleotide polymer, in linear or circular conformation, and in either single- or double-stranded form. For the purposes of the present disclosure, these terms are not to be construed as limiting with respect to the length of a polymer. The terms can encompass known analogues of natural nucleotides, as well as nucleotides that are modified in the base, sugar and/or phosphate moieties (e.g., phosphorothioate backbones). In general, an analogue of a particular nucleotide has the same base-pairing specificity; i.e., an analogue of A will base-pair with T.

The terms "polypeptide," "peptide" and "protein" are used interchangeably to refer to a polymer of amino acid residues. The term also applies to amino acid polymers in which one or more amino acids are chemical analogues or modified derivatives of a corresponding naturally-occurring amino acids.

The term "sequence" refers to a nucleotide sequence of any length, which can be DNA or RNA; can be linear, circular or branched and can be either single-stranded or double stranded. The term "donor sequence" refers to a nucleotide sequence that is inserted into a genome. A donor sequence can be of any length, for example between 2 and 10,000 nucleotides in length (or any integer value therebetween or there above), preferably between about 100 and 1,000 nucleotides in length (or any integer therebetween), more preferably between about 200 and 500 nucleotides in length.

### EXAMPLES

### EXAMPLE 1

### Batches

Pre-clinical (R&D), Engineering (Eng Run) and GMP (GMP Run) batches may be three batches of lentiviral vector obtained from LENTIGEN. Engineering batch may be similar to the GMP batch (32L each, but QC control and release specifications may be more stringent for the GMP batch). Pre-clinical batches (not shown) may be smaller vector batch preparations (4L) than Eng Run and GMP Run. Titers, for example, for Pre-clinical batch, Engineering batch, and GMP batch may be 1.1×10⁹ TU/ml, 1.9×10⁹ TU/ml, and 8.3 ×10⁹ TU/ml, respectively.

### Similar transduction efficiency between different batches based on volumetric concentrations

Transduction of T cells from healthy donors with a lentiviral vector expressing R7P1D5 TCR (LV-R73) may be performed by activating T cells with immobilized anti-CD3/anti-CD28 antibodies (Invitrogen) where the cells are cultured in TexMACS media (Miltenyi), 5% human AB serum with IL-7 and IL-15 for expansion, provided that the cytokine(s) is not IL-2 alone, not IL-7 alone, not a combination of IL-2, IL-7, and IL-15, or not a combination of IL-2 and IL-7. This procedure preserved an early T cell differentiation phenotype (T naïve/scm: CD45RA+CCR7+ or CD45RA+CCR7+CD62L+ or CD45RO-CCR7+ or CD45RO-CCR7+CD62L+), and the transduced cells showed proliferation comparably to non-transduced T cells. Other cytokines, such as IL-10, IL-12, IL-21, interferons, and TGF-β, may also be used. The transduced cells may also show CD28+CD27+ phenotypes.

### Copy number

Copy number refers to the quantification of the proviral vector genomes in the extracted genomic DNA from the T cell product and their normalization by the quantity of a reference gene of a known copy number. The most commonly used method to determine copy number is a quantitative real-time PCR (qPCR) based on standard TaqMan platform (Applied Systems). Thus, copy number may refer to the average number of the integrated vector sequences detected in the genomic DNA isolated from the T cell product using quantitative PCR. Genomic DNA may be isolated from the T cells and amplified using primer/probe assay specific for the lentiviral vector and house-keeping gene. For example, copy number determination qPCR assay may be used to quantify lentivector integrated genomes relative to an endogenous reference gene (Albumin). Efficacy and safety of T cells products are determined by average copy number. Higher integration generally results in higher transgene expression but also increases the risk of insertional mutagenesis. FDA regulations describe an average copy number of 5 or less as a safety specification for cellular products. Therefore, copy number may be considered as a critical parameter in determining the potency of a LV batch and the optimal volumetric concentration for clinical manufacturing.

Cell concentration during transduction was fixed at about 2 × 10⁶ cells/ml. FIGS. 1, 3, and 5 (solid lines) show primary T cells obtained from 3 healthy donors, i.e., donor 6, donor 7, and donor 9, respectively, transduced with increasing volumetric concentrations of lentivirus (LV)-R73, exhibit increasing and comparable copy numbers of integrated LV-R73 between engineering batches (Eng Run) and GMP batches (GMP Run) in a volumetric concentration (e.g., 3 fold serial dilution plotted on a log scale)-dependent manner of LV-R73 per 1.0 ml of transduction mixture, which may contain about 2 × 10⁶ cells.

In contrast, as shown in FIGS. 2, 4, and 6 (solid lines), significant differences in copy numbers of integrated LV-R73 between Eng Run and GMP Run were obtained from donor 6, donor 7, and donor 9, respectively, with increasing multiplicities of infection (MOI).

These results show that based on volumetric concentrations comparable copy numbers of integrated lentiviral vector were obtained between two vector manufacturing batches of LV-R73 (Engineering batch and GMP batch).

### Transgene expression

Transgene expression refers to detection of transgenic TCR on T cell surface by flow-cytometry using specific HLA-dextramer. Results are expressed as percentage of Dextramer+ve cells of the total CD3+CD8+ cells (% of Dex+).

T cells transduced by LV-R73 were tested for binding to a MHC HLA-A2-peptide dextramer. The MHC Dextramer (Dex) may contain a dextran polymer backbone carrying an optimized number of MHC and fluorochrome molecules. MHC Dextramer reagents carry more MHC molecules and more fluorochromes than conventional MHC multimers. This increases avidity for the specific T cell and enhances staining intensity, thereby increasing resolution and the signal-to-noise ratio. For staining, the protocol supplied by the manufacturer was followed. Samples were acquired on MACS Quant Analyzer (Miltenyi), and data were analyzed by Flow Jo software.

FIGS. 1, 3, and 5 (dotted lines) show primary T cells obtained from 3 healthy donors, i.e., donor 6, donor 7, and donor 9, respectively, transduced with LV-R73, exhibit increasing and comparable levels of transgene, e.g., R73 TCR, expression (% of Dex⁺) between large-scale engineering batches (Eng Run) and GMP batches (GMP Run) in a volumetric concentration (e.g., 3-fold serial dilution plotted on a log scale)-dependent manner of LV-R73 per 1.0 ml of transduction mixture, which may contain about 2 × 10⁶ cells.

However, when comparing % of Dex+ cells between Eng run and GMP Run based on multiplicities of infection (MOI) values determined by viral titers, the two batches (Eng Run and GMP run) differed significantly, as shown in FIGS. 2, 4, and 6 (dotted lines).

These results show that, based on volumetric concentrations, two vector manufacturing batches of LV-R73 (Engineering batch and GMP batch) produce comparable transgene expression (% Dextramer+ve of CD3+CD8+ cells) in primary T cells derived from healthy donors.

These results suggest that volumetric method may be more reliable than multiplicities of infection (MOI) method that relies on the virus titers determined e.g., using HEK293T cells, to compare the potency of different vector batches and to determine the optimal vector volume for clinical manufacturing of the genetically modified products in advanced stage clinical trials that require use of more than one vector batch.

### Optimization of viral volumetric concentration for T cell transduction

Following broad range comparison, as shown in FIGS. 1-6, a few volumetric concentrations are selected falling for further screening in primary T cells obtained from multiple healthy donors. Small-, mid-, and large-scale T cell manufacturing runs were conducted using selected volumetric concentrations, e.g., 5 µl, 7.5 µl, and 10 µl.

Cell concentration during transduction was fixed at about 2 × 10⁶ cells/ml. Primary T cells obtained from 10 healthy donors were transduced with LV-R73 at increasing viral volumetric concentrations, e.g., 5 µl, 7.5 µl, and 10 µl of LV-R73 per 0.5 ml of transduction mixture, which may contain about 1 × 10⁶ cells. Transgene expression and integration copy number of viral vector, e.g., LV-R73, were determined, as described above, at 8 days and 10 days post-transduction.

FIG. 7 shows average transgene expression (%Dex⁺) in transduced T cells from 10 donors (open and solid squares) increases in a volumetric concentration-dependent manner. That is, 5 µl of LV-R73 per 0.5 ml of transduction mixture yielded the lowest average quantity of the transduced T cells that express the transgene and 10 µl of LV-R73 per 0.5 ml of transduction mixture resulted in the highest average quantity of the transduced T cells that express the transgene. In addition, average quantity of the transduced T cells that express the transgene do not change significantly from 8 days post-transduction to 10 days post-transduction at each volumetric concentration.

FIG. 8 shows average integration copy numbers (Copy #) of viral vector, e.g., LV-R73, in transduced T cells from 10 donors (open and solid squares) increase in a volumetric concentration-dependent manner. That is, 5 µl of LV-R73 per 0.5 ml of transduction mixture yielded the lowest average integration copy number of viral vector and 10 µl of LV-R73 per 0.5 ml of transduction mixture resulted in the highest average integration copy number of viral vector. However, average integration copy number decreases from 8 days post-transduction to 10 days post-transduction at each volumetric concentration. These results show that, although average integration copy number decreases at 10 days post-transduction, average quantity of the transduced T cells that express the transgene remain comparable to that at 8 days post-transduction.

These results show viral volumetric concentration consistently showing maximum % HLA-Multimer+ve cells across different donors and scales without exceeding the copy number of 5 may be selected as optimal viral volumetric concentration for clinical manufacturing.

FIG. 9 shows a method (90) of transducing T cells for immunotherapy according to one embodiment of the present disclosure including obtaining T cells from a plurality of healthy donors (91), activating the T cells with an anti-CD3 antibody and an anti-CD28 antibody (92), transducing the activated T cells with a viral vector at a plurality of volumetric concentrations (93), expanding the transduced T cells (94), e.g., for 4-6 days, measuring a quantity of the expanded T cells that express the transgene and/or copy number of integrated transgene in the expanded T cells at the plurality of volumetric concentrations (95), identifying the volumetric concentration that yields a maximum average quantity of the transduced T cells that express the transgene and/or a maximum average copy number of integrated transgene in each of the expanded T cells from the plurality of healthy donors (96), and transducing T cells obtained from a patient with the viral vector at the identified volumetric concentration for the immunotherapy (97).

Advantages of the present disclosure may include methods that can accurately define quantity of lentiviral vector used during production of the T cell product using the same process as used in manufacturing of the T cell product. Because there is no relationship between virus titer and volumetric concentration, the volumetric concentration method is advantageous over the multiplicities of infection (MOI) method that relies on the virus titers. As such, methods of the present disclosure may be more robust than conventional methods that use cell line-derived virus titers to obtain optimal multiplicities of infection (MOI) and can reduce variations when multiple lentiviral vector batches are required for the manufacturing of the same T cell product.

## Claims

1. A method of making T cells suitable for immunotherapy, comprising
(A1) obtaining primary T cells from at least one healthy individual;
(A2) activating the T cells obtained in step (A1) with an anti-CD3 antibody and an anti-CD28 antibody;
(A3) transducing the activated T cells of step (A2) with a transduction mixture comprising a cell concentration of 0.1 × 10⁶ cells/ml to 1.0 × 10⁸ cells/ml and a retroviral vector at a plurality of volumetric concentrations; viz. volume of virus sample per volume of transducing mixture, wherein the retroviral vector carries a transgene;
(A4) expanding the transduced T cells of step (A3), and
(A5) identifying the volumetric concentration of the retroviral vector that yields a maximum average of the quantity of the expanded T cells that express the transgene and/or a maximum average of the copy number of the integrated transgene without exceeding five copies of the integrated transgene in each of the expanded T cells from step (A4);
and
(B1) obtaining primary T cells from a patient;
(B2) activating the T cells obtained in step (B1) with an anti-CD3 antibody and an anti-CD28 antibody;
(B3) transducing the activated T cells of step (B2) with a transduction mixture comprising a cell concentration of 0.1 × 10⁶ cells/ml to 1.0 × 10⁸ cells/ml and the retrovirus used in step (A3) at the retroviral volumetric concentration identified in step (A5);
(B4) expanding the transduced primary T cells of step (B3).

2. The method of claim 1, wherein the retroviral vector expresses a T cell receptor (TCR).

3. The method of claim 1 or 2, wherein the retroviral vector is a lentiviral vector expressing a TCR.

4. The method of any one of claims 1 to 3, wherein the transduction mixture comprises a cell concentration of from about 0.1 × 10⁶ cells/ml to about 1.0 × 10⁶ cells/ml, from about 0.5 × 10⁶ cells/ml to about 1.0 × 10⁶ cells/ml, from about 1.0 × 10⁶ cells/ml to about 1.0 × 10⁷ cells/ml, from about 5.0 × 10⁶ cells/ml to about 1.0 × 10⁷ cells/ml, from about 1.0 × 10⁷ cells/ml to about 1.0 × 10⁸ cells/ml, or from about 5.0 × 10⁷ cells/ml to about 1.0 × 10⁸ cells/ml.

5. The method of any one of claims 1 - 4, wherein the T cells in step (A1) are obtained from a plurality of healthy individuals.

6. The method of any one of claims 1-5, wherein the patient has cancer.

7. The method of claim 6, wherein the cancer is selected from the group consisting of hepatocellular carcinoma (HCC), colorectal carcinoma (CRC), glioblastoma (GB), gastric cancer (GC), esophageal cancer, non-small cell lung cancer (NSCLC), pancreatic cancer (PC), renal cell carcinoma (RCC), benign prostate hyperplasia (BPH), prostate cancer (PCA), ovarian cancer (OC), melanoma, breast cancer, chronic lymphocytic leukemia (CLL), Merkel cell carcinoma (MCC), small cell lung cancer (SCLC), Non-Hodgkin lymphoma (NHL), acute myeloid leukemia (AML), gallbladder cancer and cholangiocarcinoma (GBC, CCC), urinary bladder cancer (UBC), acute lymphoblastic leukemia (ALL), and uterine cancer (UEC).

8. The method of any one of claims 1-7, wherein the T cells obtained in step (A1) and step (B1) are CD8⁺ T cells.

9. The method according to anyone of claims 1-8, wherein the expanding in step A4 and B4 is in the presence of IL-7, IL-10, IL-12, IL-15, and IL-21, provided that the activating is not in the presence of IL-7 alone.

10. The method according to claim 9, wherein the transduced T cells exhibit a phenotype of CD45RA+CCR7+ or CD45RA+CCR7+CD62L+ or CD45RO-CCR7+ or CD45RO-CCR7+CD62L+.

11. The method according to claim 9, wherein the transduced T cells exhibit a phenotype of CD28+CD27+.

## Patentansprüche

1. Verfahren, um T-Zellen für die Immuntherapie geeignet zu machen, umfassend:
(A1) Erhalten von primären T-Zellen von mindestens einem gesunden Individuum;
(A2) Aktivieren der T-Zellen, welche im Schritt (A1) erhalten wurden, mit einem Anti-CD3 Antikörper und einem Anti-CD28 Antikörper;
(A3) Transduktion von T-Zellen des Schritts (A2) mit einer Transduktionsmischung umfassend eine Zellkonzentration von 0,1 × 10⁶ Zellen/ml bis 1,0 × 10⁸ Zellen/ml und einen retroviralen Vektor in einer Mehrzahl von volumetrischen Konzentrationen; nämlich Volumen von einem Virusmuster per Volumen der Transduktionsmischung, wobei der retrovirale Vektor ein Transgen trägt;
(A4) Expansion der transduzierten T-Zellen des Schrittes (A3), und
(A5) Identifizieren der volumetrischen Konzentration des retroviralen Vektors, die einen maximalen Durchschnitt der Menge der expandierten T-Zellen ergibt, welche das Transgen und/oder einen maximalen Durchschnitt der Anzahl an Kopien des integrierten Transgens exprimieren, ohne fünf Kopien zu überschreiten des integrierten Transgens in jeder der expandierten T-Zellen aus dem Schritt (A4);
und
(B1) Erhalten von primären T-Zellen von einem Patienten;
(B2) Aktivieren der T-Zellen welche im Schritt (B1) erhalten wurden, mit einem Anti-CD3 Antikörper und einem Anti-CD28 Antikörper;
(B3) Transduktion von aktivierten T-Zellen des Schritts (B2) mit einer Transduktionsmischung umfassend eine Zellkonzentration von 0,1 × 10⁶ Zellen/ml bis 1,0 × 10⁸ Zellen/ml und einen retroviralen Vektor, welcher im Schritt (A3) verwendet wurde, bei einer im Schritt (A5) festgelegten volumetrischen Konzentration des Retrovirus;
(B4) Expansion der transduzierten primären T-Zellen des Schrittes (B3).

2. Verfahren nach Anspruch 1, wobei der retrovirale Vektor einen T-Zell-Rezeptor (TCR) exprimiert.

3. Verfahren nach Anspruch 1 oder 2, wobei es sich bei dem retroviralen Vektor um einen lentiviralen Vektor handelt, der einen TCR exprimiert.

4. Das Verfahren nach einem der Ansprüche 1 bis 3, wobei die Transduktionsmischung eine Zellkonzentration von ungefähr 0,1 × 10⁶ Zellen/ml bis ungefähr 1,0 × 10⁶ Zellen/ml, von ungefähr 0,5 × 10⁶ Zellen/ml bis ungefähr 1,0 × 10⁶ Zellen/ml, von ungefähr 1,0 × 10⁶ Zellen/ml bis ungefähr 1,0 × 10⁷ Zellen/ml, von ungefähr 5,0 × 10⁶ Zellen/ml bis ungefähr 1,0 × 10⁷ Zellen/ml, von ungefähr 1,0 × 10⁷ Zellen/ml bis ungefähr 1,0 × 10⁸ Zellen/ml, oder von ungefähr 5,0 × 10⁷ Zellen/ml bis ungefähr 1,0 × 10⁸ Zellen/ml umfassen.

5. Verfahren nach einem der Ansprüche 1 - 4, wobei die T-Zellen im Schritt (A1) von einer Vielzahl von gesunden Individuen erhalten werden.

6. Verfahren nach einem der Ansprüche 1 - 5, wobei der Patient Krebs hat.

7. Verfahren nach Anspruch 6, wobei der Krebs aus der Gruppe bestehend aus hepatozellulärem Karzinom (HCC), Darmkrebs (CRC), Glioblastom (GB), Magenkrebs (GC), Speiseröhrenkrebs, nicht-kleinzelligem Lungenkrebs (NSCLC), Bauchspeicheldrüsenkrebs (PC), Nierenzellkarzinom (RCC), gutartiger Prostatahyperplasie (BPH), Prostatakrebs (PCA), Eierstockkrebs (OC), Melanom, Brustkrebs, chronischer lymphatischer Leukämie (CLL), Merkelzellkarzinom (MCC), kleinzelligem Lungenkrebs (SCLC), Non-Hodgkin-Lymphom (NHL), akuter myeloischer Leukämie (AML), Gallenblasenkrebs und Cholangiokarzinom (GBC, CCC), Harnblasenkrebs (UBC), akuter Lymphoblastenleukämie (ALL) und Gebärmutterkrebs (UEC) ausgewählt ist.

8. Verfahren nach einem der Ansprüche 1-7, wobei die im Schritt (A1) und im Schritt (B1) erhaltenen T-Zellen CD8⁺ T-Zellen sind.

9. Verfahren nach einem der Ansprüche 1-8, wobei die Expansion im Schritt (A4) und (B4) in Gegenwart von IL-7, IL-10, IL-12, IL-15 und IL-21 erfolgt, unter der Voraussetzung, dass die Aktivierung nicht in Gegenwart von IL-7 allein erfolgt.

10. Verfahren nach Anspruch 9, wobei die transduzierten T-Zellen einen Phänotyp von CD45RA+CCR7+ oder CD45RA+CCR7+CD62L+ oder CD45RO-CCR7+ oder CD45RO-CCR7+CD62L+ aufweisen.

11. Verfahren nach Anspruch 9, wobei die transduzierten T-Zellen einen Phänotyp von CD28+CD27+ aufweisen.

## Revendications

1. Procédé de production de lymphocytes T appropriés pour l'immunothérapie, comprenant :
(A1) l'obtention de lymphocytes T primaires d'au moins un individu en bonne santé ;
(A2) l'activation des lymphocytes T obtenus à l'étape (A1) avec un anticorps anti-CD3 et un anticorps anti-CD28 ;
(A3) la transduction des lymphocytes T activés de l'étape (A2) avec un mélange de transduction comprenant une concentration cellulaire de 0,1 × 10⁶ cellules/ml à 1,0 × 10⁸ cellules/ml et un vecteur rétroviral à diverses concentrations volumétriques ; à savoir volume d'échantillon viral par volume de mélange de transduction, où le vecteur rétroviral transporte un transgène ;
(A4) l'amplification des lymphocytes T transduits de l'étape (A3), et
(A5) l'identification de la concentration volumétrique du vecteur rétroviral qui génère une quantité moyenne maximale de lymphocytes T amplifiés exprimant le transgène et/ou une nombre moyen maximal de copies du transgène intégré sans dépasser cinq copies du transgène intégré dans chacun des lymphocytes T amplifiés de l'étape (A4) ;
et
(B1) l'obtention des lymphocytes T primaires d'un patient ;
(B2) l'activation des lymphocytes T obtenus à l'étape (B1) avec un anticorps anti-CD3 et un anticorps anti-CD28 ;
(B3) la transduction des lymphocytes T activés de l'étape (B2) avec un mélange de transduction comprenant une concentration cellulaire de 0,1 × 10⁶ cellules/ml à 1,0 × 10⁸ cellules/ml et le rétrovirus utilisé à l'étape (A3) à la concentration volumétrique rétrovirale identifiée à l'étape (A5) ;
(B4) l'amplification des lymphocytes T primaires transduits de l'étape (B3).

2. Procédé de la revendication 1, où le vecteur rétroviral exprime un récepteur des lymphocytes T (TCR).

3. Procédé de la revendication 1 ou 2, où le vecteur rétroviral est un vecteur lentiviral exprimant un TCR.

4. Procédé de l'une quelconque des revendications 1 à 3, où le mélange de transduction comprend une concentration cellulaire d'environ 0,1 × 10⁶ cellules/ml à environ 1,0 × 10⁶ cellules/ml, d'environ 0,5 × 10⁶ cellules/ml à environ 1,0 × 10⁶ cellules/ml, d'environ 1,0 × 10⁶ cellules/ml à environ 1,0 × 10⁷ cellules/ml, d'environ 5,0 × 10⁶ cellules/ml à environ 1,0 × 10⁷ cellules/ml, d'environ 1,0 × 10⁷ cellules/ml à environ 1,0 × 10⁸ cellules/ml ou d'environ 5,0 × 10⁷ cellules/ml à environ 1,0 × 10⁸ cellules/ml.

5. Procédé de l'une quelconque des revendications 1 à 4, où les lymphocytes T de l'étape (A1) sont obtenus de divers individus en bonne santé.

6. Procédé de l'une quelconque des revendications 1 à 5, où le patient présente un cancer.

7. Procédé de la revendication 6, où le cancer est sélectionné à partir du groupe de cancers suivants : carcinome hépatocellulaire (CHC), carcinome colorectal (CCR), glioblastome (GB), cancer gastrique (CG), cancer de l'œsophage, cancer du poumon non à petites cellules (CPNPC), cancer du pancréas (CP), carcinome rénal (CR), hyperplasie bénigne de la prostate (HBP), cancer de la prostate (CAP), cancer de l'ovaire (CO), mélanome, cancer du sein, leucémie lymphoïde chronique (LLC), carcinome à cellules de Merkel (CCM), cancer du poumon à petites cellules (CPPC), lymphome non hodgkinien (LNH), leucémie aiguë myéloïde (LAM), cancer de la vésicule biliaire et cholangiocarcinome (CVB, CCC), cancer de la vessie urinaire (CVU), leucémie aiguë lymphoblastique (LAL) et cancer de l'utérus (CU).

8. Procédé de l'une quelconque des revendications 1 à 7, où les lymphocytes T obtenus à l'étape (A1) et à l'étape (B1) sont des lymphocytes T CD8⁺.

9. Procédé conforme à l'une quelconque des revendications 1 à 8, où l'amplification des étapes A4 et B4 se fait en présence d'IL-7, IL-10, IL-12, IL-15 et IL-21, à condition que l'activation ne se fasse pas en présence d'IL-7 seule.

10. Procédé conforme à la revendication 9, où les lymphocytes T transduits présentent un phénotype CD45RA+CCR7+ ou CD45RA+CCR7+CD62L+ ou CD45RO-CCR7+ ou CD45RO-CCR7+CD62L+.

11. Procédé conforme à la revendication 9, où les lymphocytes T transduits présentent un phénotype CD28+CD27+.
